# EUROPEAN PATENT APPLICATION

(11) **EP 1 022 278 A1**
(43) Date of publication of application: **26.07.2000**
(21) Application number: 97930907.7
(22) Date of filing: 02.04.1997
(51) Int. Cl.: C07D 513/14, A61K 31/54

(54) **TETRACYCLICAL DERIVATIVES FROM PYRIMIDINE**

(71) Applicant: Ashkinazi, Rimma Iliinichna, St. Petersburg, 191025 (RU)
(72) Inventor: ASHKINAZI, Rimma Iliinichna, St.Petersburg, 191025 (RU); KRASNOV, Konstantin Andreevich, St.Petersburg, 199155 (RU)
(74) Representative: Mercer, Christopher Paul
(86) International application number: PCT/RU97/00098
(87) International publication number: WO 98/43982

(57) **Abstract**

Biologically active substance on the basis of tetracyclic nitrogen heterocycles of pyrimidine series (applicant - inventors R.I. Ashkinazi and K.A. Krasnov). The present invention relates to medicine, and more specifically to pharmacology, veterinary cosmetology, and in particular to synthetic biologically active compounds of a heterocycle series, which possess antimicrobial, antiviral, antichlamydia and immune stimulating activities: derivatives of 5-oxo-5H-[1]-benzopyrano-[5,6-b]-4-oxo-4H-[1,2]-pyrimido-1,4,5,64-tetrahydro-1,3-thiazine. The compounds are provided mainly for treating tuberculosis, mycobacteriosis, viral diseases, infections caused by chlamydias, and diseases which are accompanied by immunodeficiency, particularly malignant neoplasm. In addition, the above mentioned compounds can be used for the same purposes in veterinary medicine and cosmetology.

An objective of the invention is to obtain new chemical compounds which have high antimicrobial activity, particularly with respect to strains of mycobacteria that are resistant to isoniazid, which is standardly used to treat mycobacteria. Compounds of the invention simultaneously possess antiviral activity (relative to herpes simplex viruses), antichlamydial activity, and also stimulate production of endogenic interferons in organisms. In other words, the objective of the invention is a chemical synthesis of biologically active substances which are superior to the prototype not only in a range of action on strains of tuberculosis and other mycobacteria, but also possess antiviral, antichylamidial and immuno stimulating action.

The objective is solved by synthesis of new class of heterocyclic compounds-derivatives of 5-oxo-5H-[1]-benzopyrano[5,6-b]-4-oxo-4H-[1,2]-pyrimido-1,4,5,6-tetrahydro-1,3-thiazine (1) of general formula (1). 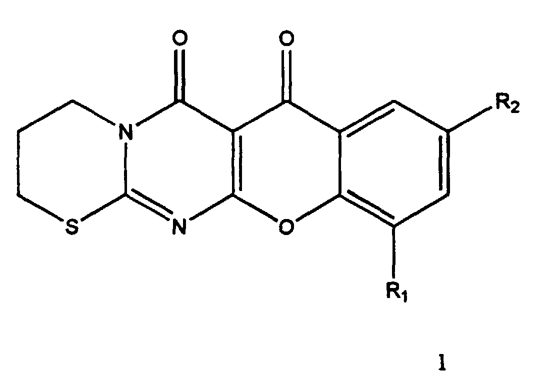 (I-X),where: R₁ = H or a halogen; R₂ = H, halogen, nitro, hydroxy, or methoxy.

The objective can be solved with R₁ = R₂ = H (I); R₁ = H and R₂ = Cl (II); R₁ = R₂ = Cl (III); R₁ = H and R₂ = Br (IV); R₁ = R₂ = Br (V); R₁ = H and R₂ = NO₂ (VI); R₁ = Cl and R₂ = NO₂ (VIIl); R₁ = Cl and R₂ = NO₂ (VII); R₁ = Br and R₂ = NO₂ (VIII); R₁ = H and R₂ = OCH₃ (IX); R₁ = H and R₂ = OH (X).

The proposed synthesis of the claimed compounds includes two main stages:
1. 4-oxo-4H-6-oxy-[1,2-d]-pyrimido-1, 4, 5, 6-tetrahydro-1,3-thiazine(XI) is synthesized from 2-thiobarbituric acid (XII) and 1,3-dihalogenopropane(XIII); and
2. A target compound (I-X) is produced from the intermediate substance (XI) obtained in the first stage and corresponding derivative of salicylaldehyde (XIV). The method is common for all members of the group.

The subject matter of the present invention it explained by two examples of synthesis of the intermediate substance and three examples of synthesis of claimed substances, two tables of yield of intermediate and target products, two tables of characteristics of target products, and data of seven experiments for determination of their biological properties.

## Description

### BACKGROUND OF THE INVENTION

The present invention relates to medicine, and more specifically to pharmacology, veterinary medicine, cosmetology, and in particular to synthetic biological active compounds of a heterocycle series, which possess antimicrobial, antiviral, antichlamydia and immune stimulating activities: derivatives of 5-oxo-5H-[1]-benzopyrano-[5,6-b]-4-oxo-4H-[1,2]-pyrimido-1,4,5,6-tetrahydro-1,3-thiazine. The derivatives mentioned above have high antimicrobial activity to various mycobacteria, substantial antiviral activity relative to *Herpes simplex virus*, high antichlamydial activity, and also activity as inducers of interferon. The compounds are intended, mainly, for treating tuberculosis, mycobacteriosis, viral diseases, infections caused by chlamydia, and also diseases which are accompanied by immunodeficiency, in particular malignant neoplasm. In addition, the above mentioned compounds can be used for the same purposes in veterinary medicine and cosmetology.

### BACKGROUND ART

As known, there are many unsolved problems in modern chemotherapy for treating infectious diseases. Among bacterial infections, one of the most serious problems is caused by tuberculosis, whose treatment is especially complicated due to high resistivity of tuberculosis mycobacteria to the action of chemotherapeutical agents [1]. Moreover, existing compounds possess a substantial disadvantage in that a wide spectrum of mycobacteria are resistant to them.

Historically, the first antituberculosis compounds were derivatives of phenols, benzoic acids (2), mercaptobenzimidizols (3) and p-aminosalicyclic acid (4). The most active antituberculosis agents were derivatives of isonicotine acid discovered at the end of the 1940's, *e.g.*, isoniazide and its analogs which are still used today, ethionamide, ethambutole, aminoglycosides, rifampicin, and fluoraquinols (1). During therapy of tuberculosis and mycobacteriosis, usually 2-3 compounds are simultaneously prescribed, which retard the appearance of resistant forms of mycobacteria, but do not solve the problems of overuse and resistance completely. Therefore, a search for new active antituberculosis agents, including those which act on forms of mycobacteria which are resistant to isoniazide, is still underway.

Viral diseases also pose problems for modem medicine which are serious. As a rule, viral infections, such as those caused by viruses of herpes group, are very difficult to treat because the inefficiency of existing compounds as well as rapid mutations in the virus which result in resistant forms (5-7). Synthetic compounds for treatment of diseases caused by viruses of herpes group including acyclovir, gancyclovir, retrovir (5) and others whose efficiency is not always sufficient.

Many problems exist during treatment of diseases caused by chlamydia, including widely spread urogenital infections (8). Chlamydia, which involves intracellular parasites, is hardly accessible to the majority of existing compounds. Also, widespread resistance to the latter caused an appearance of a great number of chronic forms of diseases. To treat chlamydia infections, compounds of the tetracycline series, macrolids and fluoroquinolones such as ciprophoxacin are used. (8)

Frequently, diseases of a viral and bacterial nature occur due to reduction of activity of an organism's immune system. Thus, the problem of developing efficient compounds for treatment of immune deficient states produced for a variety of reasons is also one of the most difficult problems. In addition, stimulation of activity of immune system contributes to the ability of an organism to resist tumoral processes, during which multiple resistance to existing treatment compounds is observed (9).

At present, an intense search is being conducted for biologically active compounds among derivatives of pyrimidine series. In particular, in the pyrimidine series, many derivatives are known which are used as medical compounds (10). Monocyclic derivatives of pyrimidine, particularly barbituric acids, possess universal antibacterial action because of antagonism with pyrimidine bases of DNA (11). Active bactericidal compounds are found between hydrazine derivatives of pyrimidine, thiopyrimidines (14), alkoxy- and amino- pyrimidines (15). Ethers of pyrimidine carboxylic acids exhibit antitumor activity (16).

Furthermore, many derivatives of barbituric acids possess substantial antiviral activity (13, 17). Derivatives [4,2-b] of pyrimidopyran possess anti-inflammatory properties (18). The same action is found in many N-aryl- and N-cyclohexyl- barbituric acids (19). All examples presented hereinabove are united by a common chemical structure, since they are monocyclic derivatives of pyrimidine (containing one pyrimidine heterocycle not conjugated with other cyclic systems).

An example of finding biological properties in three cyclic derivatives of pyrimidine series is known, but their action is limited by anti-allergic activity (20) and is not extended to the area of antimicrobial and antiviral action. Derivatives of 2, 4-[1H, 3H, 5H]-[1] benzopyrano [2,3-d]-pyrimidine, patented as anti-allergic agents, are the closest to the claimed compounds with respect to chemical structure (20).

For example, hydrazide of isonicotinic acid (isoniazide) has been selected (9). The selection is based on its coincidence with the claimed substance as to an important objective: anti-tuberculosis action. Isoniazide is one of the most efficient anti-tuberculosis compounds, and can be used in any medicinal form (pills, injections, inhalations). At the same time, the object of the invention and the prototype belong to the class of nitrogen heterocycles as to their chemical structure.

### OBJECTIVE OF THE INVENTION

An objective of the invention is to obtain new chemical compounds which have high antimicrobial activity, particularly with respect to strains of mycobacteria which are resistant to, for example, isoniazide, and simultaneously possess antiviral activity (relative to herpes simplex viruses), and antichlamydial activity. Novel compounds of the invention also stimulate production of endagenic interferons in organisms. In other words, the objective of the invention is a chemical synthesis of biologically active substances that are superior to isoniazide not only in a range of action on strains of tuberculosis and other mycobacteria, but also possess antiviral, antichlamydial and immunostimulating action.

### SUBJECT OF THE INVENTION

The object of the invention is solved by synthesis of a new class of heterocyclic compounds - 5-oxo-5H-[1]-benzopyrano [5,6-b]4-oxo-4H-[1,2]-pyrimido-1,4,5,6-tetrahydro-1,3-thiazine (1) derivatives of general formula (1) 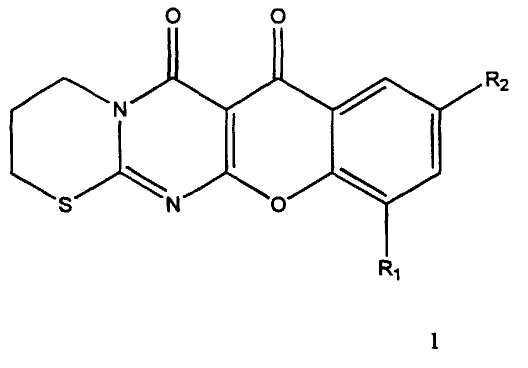 wherein: R₁ = H or halogen; R₂ = H, halogen, nitro, hydroxy, or methoxy.

The objective of the invention can be resolved at R₁ = R₂ =H(I); R₁ = H and R₂ = Cl (II); R₁ = R₂ = Cl (III); R₁ = H and R₂ = Br (IV); R₁ = R₂ = Br (V); R₁ = H and R₂ = NO₂ (VI); R₁ = Cl and R₂ = N0₂ (VII); R₁ = Br and R₂ = N0₂ (VIII); R₁ = H and R₂ = OCH₃ (IX); and R₁ = H and R₂ = OH (X).

The claimed compounds are new since they are not known from available sources of information.

Furthermore, the claimed compounds are not obvious. It is well known that it is not possible to predict in advance the biological activity of new condensed heterocyclic systems. Therefore, to obtain a group of claimed compounds which are complicated tetracyclic structures with four heteroatoms (two atoms of nitrogen, one atom of oxygen and one atom of sulfur), and to find their antimicrobal, immunostimulating and antiviral properties, is not derived in an obvious manner from the modern state of art.

### DISCLOSURE OF INVENTION

The synthesis of the claimed substances proposed by us includes two main stages:
1. 4-oxo-4H-6-oxy-[1,2-d]-pyrimido-1,4,5,6-tetrahydro-1,3-thiazine (XI) is synthesized from 2-thiobarbituric acid (XII) and 1,3-dihalogenopropane (XIII); and
2. A target compound (I-X) is produced from the intermediate substance (XI) obtained in the first stage and a corresponding derivative of salicylaldehyde (XIV). The method is common for all members of the group.

The subject matter of the present invention is explained by two examples of synthesis of the intermediate substance and three examples of synthesis of claimed substances, as well as two tables *infra* with data regarding the yields of intermediate and target products, two tables *infra* of characteristics of target product, and data *infra* from seven experiments for determination of their biological properties, where:

Examples 1 and 2 are concrete variants of execution of the first stage of synthesis of the target substance (producing of intermediate substance (XI) or in other words 4-oxo-4H-6-oxy-[1, 2-d]-pyrimido-1,4,5,6-tetrahydro-1,3-thiazine, and Example 2 contains generalized multiple variant;

Examples 3, 4, and 5 are concrete variants of execution of second stage of synthesis of claimed substances (production of target products (I-X)), or in other words, derivatives 5-oxo-5H-[1]-benzopyrano-[5,6-b]-4-oxo-4H-[1,2]-pyrimido-1,4,5,6-tetrahydro-1,3-thiazine.

Table 1 contains data of yield of intermediate substance (XI), *e.g.* 4-oxo-4H-6-oxy-[1,2-d]-pyrimido-1,4,5,6-tetrahydro-1,3-thiazine.

Table 2 shows methods of synthesis and contains data of yield of target products (I-X) synthesised by different methods, *e.g.*, derivatives 5-oxo-5H-[1]-benzopyrano-[5,6-b]-4-oxo-4H-[1,2]-pyrimido-1,4,5,6-tetrahydro-1,3-thiazine.

Table 3 contains the melting points and NMR ¹H spectra data of target products (I-X), or in other words, derivatives 5-oxo-5H-[1]-benzopyrano-[5,6-b]-4-oxo-4H-[1,2]-pyrimido-1,4,5,6-tetrahydro-1,3-thiazine.

Table 4 shows data of elementary analysis of target products (I-X), or in other words derivatives 5-oxo-5H-[1]-benzopyrano-[5,6-b)-4-oxo-4H-[1,2]-pyrimido-1,4,5,6-tetrahydro-1,3-thiazine.

Data of 7 series of experiments for determining biological activity of claimed compounds contain:
Experiment 1 - determination of antimicrobial action;
Experiment 2 - determination of action of claimed compounds on pathogenic mycobacteria;
Experiment 3 - determination of acute toxicity;
Experiment 4- therapeutic efficiency during experimental tuberculosis;
Experiment 5 - determination of action on herpes virus;
Experiment 6 - determination of interferon-inducing activities; and
Experiment 7 - determination of action on *C. trachomatis*.

### Example 1: Variant of execution of the first stage of synthesis of claimed compounds (producing of intermediate (XI), or in other words 4-oxo-4H-6-oxy-[1,2-d]-pyrimido-1, 4,5,6-tetrahydro-1,3-thiazine) (XI)

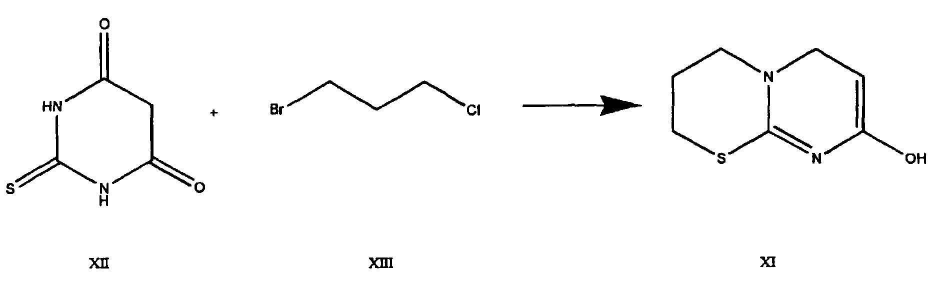

14.4 g (0.1 mol) 2-thiobarbituric acid (XII) is added to 100-150 ml of water-alcohol solution of 0.2 mol alkali and mixed. Then 0.1 mol of 1-bromo-3-chloropropanc (XIII) is added, and the mixture is mixed with heating. In the process of reaction, the pH of the solution is reduced. Alter the reaction, the solution is cooled and the precipitated residue is separated and washed. For purifying, the product is dissolved in aqueous alkali and precipitated with hydrochloric acid. Yield of 4-oxo-4H-6-oxy-[1,2-d)-pyrimido-1,4,5,6-tetrahydro-1,3-thiazine having a melting point of 250-260°C (with deviation), is 9.7g (43.8%).

The experimental and calculated elementary analysis of the compound is set forth below:

| | | | | |
|---|---|---|---|---|
| Experimental %: | C 45.02; | H 5.27; | N 14.09; | S 17.11; (C₇H₈N₂0₂S). |
| Calculated %: | C 45.07; | H 5.21; | N 14.15; | S 17.02 |

NMR ¹H spectrum, (DMSO-d6, δ, δ ppm): 2.18, m (2H,, CH₂); 3.19 t (2H, SCH²); 3.90 t(2H, NC H₂); 5.08 s (1H, CH); 11.06 s(1H, 0H).

### Example 2: Variant of execution of the first stage of synthesis of claimed compounds (producing of intermediate XI or in other words 4-oxo-4H-6-oxy-[1,2-d]-pyrrido-1,4,56-tetrahydro-1,3-thiazine (XI)).

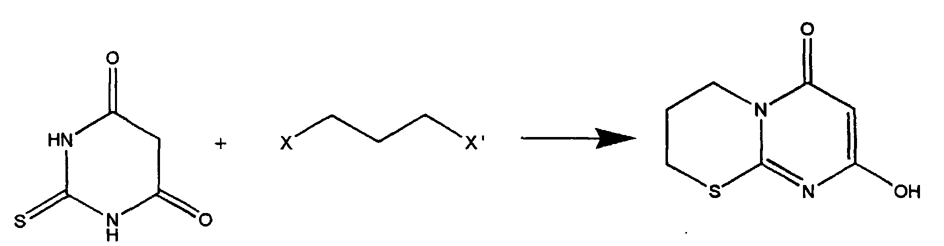

Alkylation of 2-thiobarbituric acid (XII) is performed by 1.3 dihalogeno derivatives of propane having a general formula of X-CH₂-CH₂-CH₂-X'(XIII) (where X, X'= Cl, Br or I). The process is performed analogously to the process set forth in Example I. Yields of the product are presented hereinbelow.

**Table 1**

| Yields of Intermediate Substance (XI) in Dependence on Nature of Haloid In Molecule of Reactant (XIII) and Concentration of Alcohol in Reaction Solution | | | |
|---|---|---|---|
| **X** | **X'** | **Concentration of alcohol, %** | **Yield of product (XI),%** |
| Cl | Cl | 50 | 11.5 |
| Br | Br | 50 | 46.1 |
| Br | I | 50 | 47.0 |
| I | I | 50 | 42.5 |
| Cl | Br | 50 | 43.8 |
| Cl | Br | 20 | 33.4 |
| Cl | Br | 80 | 39.6 |

### Example 3: Involves a variation of Stage two of the synthesis of the claimed substance (producing of target product, in particular 5-oxo-5H-[1]-benzopyrano-[5,6-b]-4-oxo-4H-[1,2]-pyrimido-1,4,5,6-tetrahydro-1,3-thiazine (1)).

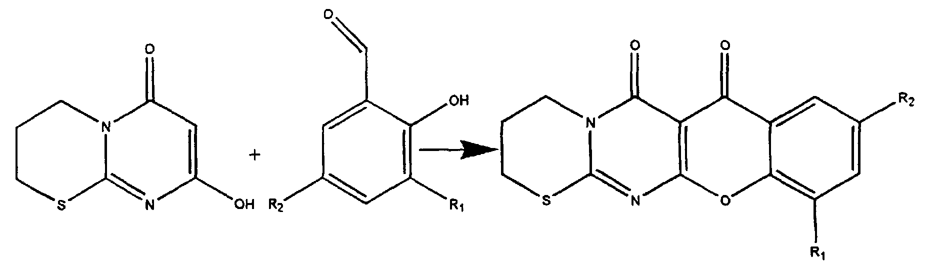

Mixture 1.84 g (0.01 mol) 4-oxo-4H-6-oxy-[1,2-d]-pyrimido-1,4,5,6-tetrahydro-1,3-thiazine (XI) and 1.83 g (0.015 mol) of salicylaldehyde (XV),(R₁ = R₂ = H) is heated, till evaporation of water vapors formed during the reaction is stopped. Then the formed reaction mass is thoroughly washed with alcohol to remove initial and side products. The residue is then recrystalized from acetic acid to obtain the target product 5-oxo-5H-f-[1]-benzopyrano-[5,6-b]-4-oxo-4H-[1,2]-pyrimido-1,4,5,6-tetrahydro-1,3-thiazine. The yield is given Table 2 and the characteristics in Table 3. Data of the element analysis of the compound are given in the Table 4.

### Example 4: Involves a variation of Stage Two of the synthesis of claimed substances (production of target product in particular 3-chloro-5-oxo-5H-[1]-benzopyrano-[5,6-b]-4-oxo-4H-[1,2]- pyrimido-1,4,5,6-tetrahydro-1,3-thiazine(II)).

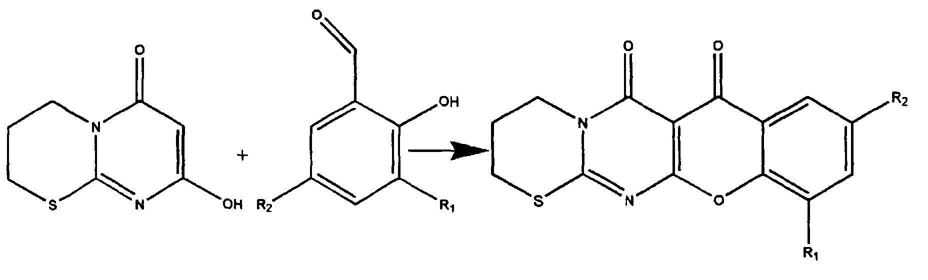

(R₁ = H, R₂ = Cl) Mixture 1.84 g (0.01 mol) 4-oxo-4H-6-oxy-[1,2-d]-pyrimido-1,4,5,6-tetrahydro-1,3-thiazine (XI) and 2.35 g (0.015 mol)-5-clorosalicylaldehyde (XV), (R₁ = H, R₂ = Cl) in highly boiling inert solvent (chlorobenzene, anizole, diglim, diethyleneglycol) is heated to disappearance of initial pyrimidothiazine. The sediment is then separated, thoroughly washed and dried. The target product obtained is 3-chloro-5-oxo-5H-[1]-benzopyrano-[5,6-b]-4-oxo-4H-[1,2]-pyrimido-1,4,5,6-tetrahydro-1,3-thiazine. The yield is given in the Table 2 and the characteristics are given in the Table 3. Data of the elementary analysis are given in the Table 4.

### Example 5: Involves a variation of Stage two of the synthesis of claimed compounds (producing of target product), in particular 1,3-dichloro-5-oxo-5H-[1]-benzopyrano-[5,6-b]-4-oxo-4H-[1,2]-pyrimido-1,4,5,6-tetrahydro-1,3- thiazine III)).

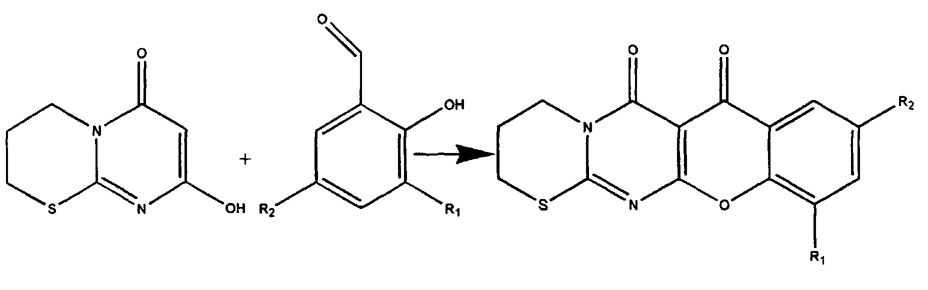

A mixture of 1.84 g (0.01 mol) 4-oxo-4H-6-oxy-[1,2-d]-pyrimido-1,4,5,6-tetrahydro-1,3-thiazine (XI) and 1.83 g (0.015 mol) of dichlorosalicylaldehyde (XV), (R₁ = R₂ = Cl), is boiled in ethanol for 6-8 hours. The precipitate that forms is filtered out of the solution, washed with hot ethanol and then dried. The target product obtained is 3-dichloro-5-oxo-5H-[1]- benzopyrano-[5,6-b]-4-oxo-4H-[1,2]-pyramido-1,4,5,6-tetrahydro-1,3-thiazine. Yield is given in Table 2, and characteristics in Table 3. Data of elementary analysis is given in Table 4. The remaining target compounds are derivatives 5-oxo-5H-[1]-benzopyrano-[5,6-b]-4-oxo-4H-[1,2]-pyrimido-1,4,5,6-tetrahydro-1,3-thiazine (IV-X), where: 3-bromo-5-oxo-5H-[1]-benzopyrano-[5,6-b]-4-oxo-4H-[1,2]-pyrimido-1,4,5,6-tetrahydro-1,3-thiazine (IV), R₁ = H, R₂ = Br; 1,3-dibromo-5-oxo-5H-[1]-benzopyrano-[5,6-b]-4-oxo-4H-[1,2]-pyrimido-1,4,5,6-tetrahydro-1,3-thiazine(V), R₁ = R₂ = Br; 3-nitro-5-oxo-5H-[1]-benzopyrano-[5,6-b]-4-oxo-4H-[1,2]-pyrimido-1,4,5,6-tetrahydro-1,3-thiazine (VI), R₁ = H, R₂ = NO₂; 1 -chloro-3-nitro-5-oxo-5H-[1]-benzopyrano-[5,6-b]-4-oxo-4H-[1,2]-pyrimido-1,4,5,6-tetrahydro-1,3-thiazine (VII) R₁ = Cl, R₂ = NO₂; 1-bromo-3-nitro-5-oxo-5H-[1]-benzopyrano-[5,6-b]-4-oxo-4H-[1,2]-pyrimido-1,4,5,6-tetrahydro-1,3-thiazine (VIII), R₁ = Br, R₂ = NO₂; 3-methoxy-5-oxo-5H-[1]-benzopyrano-[5,6-b]-4-oxo-4H-[1,2]-pyrimido-1,4,5,6-tetrahydro-1,3-thiazine (IX), R₁ = H, R₂ = -OCH₃; 3-hydroxy-5-oxo-5H-[1]-benzopyrano-[5,6-b]-4-oxo-4H-[1,2]-pyrimido-1,4,5,6-tetrahydro-1,3-thiazine (X), R₁ = H, R₂ = OH; are produced from 4-oxo-4H-6-oxy-[1,2-d]-pyrimido-1,4,5,6-tetrahydro-1,3-thiazine (XI) and corresponding salicylaldehyde derivative (XV) in accordance with the Examples 3, 4 and 5 presented above. The methods of synthesis and yields of products are given in Table 2, the characteristics in Table 3, and the data of the elementary analysis in Table 4.

**Table 2**

| Methods of Synthesis and Yield of Target Product (I-X) | | | | |
|---|---|---|---|---|
| **No Compounds** | **R**_{**1**} | **R**_{**2**} | **Method of synthesis (No of Example)** | **Yield %** |
| I | H | H | 3 | 41.0 |
| I | H | H | 4 | 38.5 |
| I | H | H | 5 | 22.4 |
| II | H | Cl | 3 | 39.9 |
| II | H | Cl | 4 | 40.4 |
| III | Cl | Cl | 3 | 44.6 |
| IV | H | Br | 3 | 42.1 |
| V | Br | Br | 4 | 35.5 |
| VI | H | NO₂ | 3 | 31.3 |
| VI | H | NO₂ | 4 | 16.2 |
| VI | H | NO₂ | 5 | 34.6 |
| VII | Cl | N0₂ | 5 | 37.5 |
| VIII | Br | N0₂ | 5 | 37.0 |
| IX | H | OCH₃ | 3 | 24.7 |
| X | H | OH | 3 | 18.3 |

**Table 3**

| Melting Points and Data of NMR ¹H Spectra of 5-oxo-5H[1]-benzopyrano-[5,6-b]-4-oxo-4H-[1,2]-pyrimido-1,4,5,6-tetrahydro-1,3-thiazine Derivative (I-X) | | | | | | | |
|---|---|---|---|---|---|---|---|
| No. Compound | T Melting Point °C | CH₂ | CH₂S | CH₂N | ArH | OCH₃(OH) | Solvent |
| I | 299 | 2.25 | 3.28 | 4.01 | 7.34 m(2H):7.71 t,8.14m | - | DMSO-d₆ |
| II | 306 | 2.26 | 3.29 | 4.06 | 7.32 m; 7.92 m; 8.22 c | - | DMSO-d₆ |
| III | 316 | 2.28 | 3.31 | 4.06 | 7.90 c; 8.11 c | - | DMSO-d₆ |
| IV | 319 | 2.29 | 3.28 | 4.08 | 7.39 m; 7.99 m; 8.30 c | - | DMSO-d₆ |
| VII | 315 | 2.39 | 3.30 | 4.22 | 8.01 c; 9.18 c | - | CDCl₃ |
| VII | 315 | 2.39 | 330 | 412 | 8.01 c; 9.18 c | - | DMSO-d₆ |
| VIII | 321 | 239 | 330 | 4.21 | 8.05 c; 9.22 c | - | DMSO-d₆ |
| IX | 292 | 226 | 3.26 | 4.09 | 7.41 m; 7.99 m; 8.54 c | 3.97 | CDCl₃ |
| X | 290 | 2.20 | 3.21 | 4.05 | 7.18 m; 7.92 m; 8.31 c | 9.12 | DMSO-d₆ |

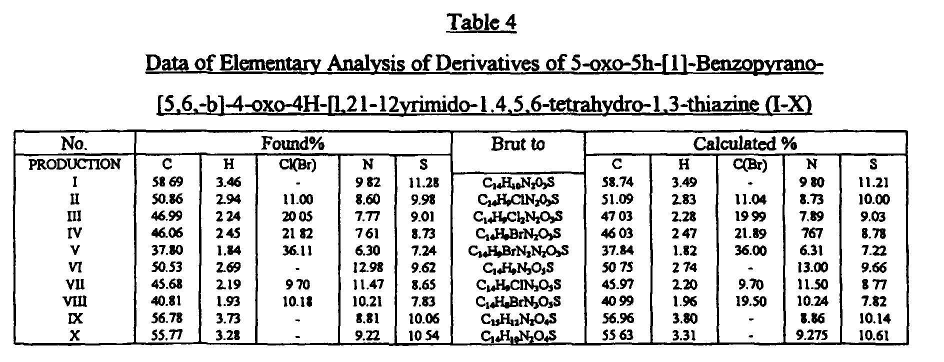

### Experimental Tests of Biological Activity of Claimed Compounds

### Experiment 1

### Determination of Antimicrobial Action of Compounds

In order to determine antimicrobial activity of compounds of the invention, standard strains of *Mycobacterium smegmatis* ATCC607 and *Mycobacterium tuberculosis* H37RV were used these strains are sensitive to all antimicrobial compounds.

The evaluation of antimicrobial action was performed with the method of series culture (5). *M. smegmatis* ATCC607 for seeding was grown on liquid synthetic medium N-1.

The compounds were dissolved in dimethyl sulfoxide (DMSO) and titred in medium N-1. As a result, the compounds were contained in separate test tubes with the medium in concentration of 200-0.025 mg/l. The concentration of the compound in the medium of neighboring test tubes was differed twice. DMSO was used as a control. It was titred in the same manner as the compound. Test strain of bacteria was added to the test tubes in the quantity of 1-2x10⁶ ml. The results were obtained after 72 hour cultivation of test tubes at 37° C.

For *M. tuberculosis* H37Rv, conditions of experiment were identical, except for the growth of the bacteria was done on the Soton medium which contains 10% horse serum, and the density of microbial suspension during seeding was (5.0 x 10⁶) in ml.

As the control, in both cases known tuberculostatic compounds were utilized. The results obtained for the used strains are summarized in Table 5.

**Table 5**

| Minimum Inhibitory Concentration (MIC) Relative to *M.tuberculosis* H37Rv and *M.smegmatis* ATCC607 (mg/l) | | | |
|---|---|---|---|
| **NN** | Compound | **MIC** | |
| | | *M. tuberculosis* | *M. smegmatis* |
| 1. | R₁ = R₂ = H | 0.6 | 0.3 |
| 2. | R₁ = H, R₂ = Cl | 10.0 | 10.0 |
| 3. | R₁ = R₂ = Cl | 0.3 | 1.0 |
| 4. | R₁ = H, R₂ = Br | 25.0 | 50.0 |
| 5. | R₁ = R₂ = Br | 0.1 | 0.1 |
| 6. | R₁ = H, R₂ = NO₂ | 50.0 | 100.0 |
| 7. | R₁ = Cl, R₂ = NO₂ | 50.0 | 100.0 |
| 8. | R₁ = Br, R₂ = NO₂ | 25.0 | 100.0 |
| 9. | R₁ = H, R₂ = OCH₃ | 50.0 | 100.0 |
| 10. | R₁ = H, R₂ = OH | 50.0 | 100.0 |

| Controls | | | |
|---|---|---|---|
| 1. | Streptomycin | 0.2 | 0.15 |
| 2. | Isoniazid | 0.1 | 1.5 |
| 3. | Rifamycin | 0.05 | 0.25 |
| 4. | Ethambutol | 5.0 | 5.0 |
| 5. | Ofloxacin | 10.5 | 10.5 |

The claimed compounds possess antibacterial activity relative to the used strains of mycobacteria in concentrations of 100.0-0.1 mg/l. The activity of action on mycobacteria of the series of claimed compounds *in vitro* is not less, and sometimes more than that of known antituberculosis compounds.

### Experiment 2

### Determination of Action of Compounds of Pathogenic Mycobacteria

In this work, the following strains taken from patients in clinics of St. Petersburg (Russia) in 1996 were utilized: *M.tuberculosis* 61 (S), which is sensitive to action of known antimicrobial compounds (aminoglycosides, rimfamycin, isoniazid, ethambutol); and *M.tuberculosis* 16 (R), which is resistant to basic antimicrobial compounds; *Mycobacterium avium* 84, mycobacteria which causes sickness and death of persons infected with Human Immunodeficiency Virus (HIV). Tests were conducted on the Löwenstein-Jensen medium. The compounds were dissolved in DMSO and added to separate test tubes with the medium in a final concentration of 10 mg/l. After seeding of test strains on the surface of the medium, the test tubes were incubated during 30 days. In the control, DMSO was used in corresponding concentration as well as standard compounds used for treatment of tuberculosis and mycobacteriosis (Table 6).

**Table 6**

| Spectrum of' Antimicrobial Action of Claimed Compounds Relative to Various Mycobacteria, Obtained from Patients (Concentration of Compound 10.0 g/l. | | | | |
|---|---|---|---|---|
| Number | Compound | *M. tuberculosis* 61S* | *M. tuberculosis* 16R** | *M.avium* 84 |
| 1. | R₁ = R₂ = H | complete growth inhibition | complete growth inhibition | complete growth inhibition |
| 2. | R₁ = R₂ = Cl | complete growth inhibition | complete growth inhibition | Complete growth inhibition |
| 3. | R₁ = R₂ = Br | complete growth inhibition | complete growth inhibition | complete growth inhibition |

| Control | | | | |
|---|---|---|---|---|
| 1. | Streptomicyn | complete growth inhibition | growth | growth |
| 2. | Isioniazid | complete growth inhibition | growth | growth |
| 3. | Rifam | complete growth inhibition | growth | growth |
| 4. | Ethambutol | complete growth inhibition | growth | partial growth inhibition |

| | | | | |
|---|---|---|---|---|
| * - sensitive to control compounds | | | | |
| ** - resistant to control compounds | | | | |

The data set forth in Table 6 clearly shows that claimed compounds of the invention inhibit the growth of all types and strains of mycobacteria used in the test. Therefore, *in vitro*, they act on strains of mycobacteria obtained from patients that are resistant to compounds standardly used to treat tuberculosis. Moreover, these compounds in the given conditions inhibit the growth of *M. avium*. The remaining claimed compounds possess lower antimicrobial activity in analogous conditions.

### Experiment 3

### Determination of Acute Toxicity

Determination of acute toxicity was performed on no-breed white mice with mass 18-20 g. Emulsions of claimed compounds were introduced in various concentrations: 1500, 700, 500, 100, 20 and 5 mg/kg. Five animals were used to test each concentration of the compound. The compound was introduced once a day orally or via intraperitoneal injection. The period of observation was 14 days. On days 1, 8 and 15, the animals of each group were weighed. For the control, animals were administered the emulsion prepared without tested compounds.

For microscopic investigation of internal organs, dissection of all animals which died during test and which survived at the end of the tests was carried out.

During the investigation weight loss, changes in behavior, changes in external appearance, as well as animal death were not observed. Based on the results of the dissection, no microscopical pathology was found in the internal organs of animals of both the test group and the control groups (Table 7).

**Table 7**

| Acute Toxicity of (LD 50) of Tested Compounds(mg/kg) | | |
|---|---|---|
| NN | Compound | LD₅₀ |
| 1. | R₁ = R₂ = H | >1500 |
| 2. | R₁ = R₂ = Cl | >1500 |
| 3. | R₁ = R₂ = Br | >1500 |

These results prove that with oral and intraperitoneal intake, the claimed compounds in concentrations 1500 mg/kg do not have acute toxicity for mice.

### Experiment 4

### Therapeutic Efficiency of Action of Claimed Compounds During Experimental Tuberculosis Process in Mice

An investigation was conducted on no-breed male mice having a mass ranging from 18 to 20 g. The tuberculosis process was caused in the mice by introduction of *M.bovis* bovinus 8 in a dose of 0.1 mg/mouse as a bacterial suspension (0.2 ml) into a tail vein of the mice. The tested compounds were used in three dosages (10, 20 and 30 mg/kg). Streptomycin was used as a compound for comparison. The tested compounds were introduced into animals once a day orally and streptomycin was introduced intramuscularly. Withdrawal of animals from the test (by decapitation) was executed at 42 day after infection, when in the control infection group (not treated mice) 45% of animals perished.

Efficiency of the therapy was evaluated in accordance with: survival of animals; relative mass of lungs; index of damage of lungs; and seeding of mycobacteria from spleen. In the latter case, homogenate of the organ was seeded on dense egg medium of Löwenstein-Jensen. A count of colonies was performed 25-30 days after cultivation. Average seeding of mycobacteria on one sample was counted, which was presented as a number of colony forming units (CFU) per 100 mg (Table 8).

**Table 8**

| Evaluation of Efficiency of Action of Tested Compounds on Infection Caused in Experimental Animals | | | | |
|---|---|---|---|---|
| Animal Group | Perished animals | Coefficient of lung mass (con. Units) | Index of damage lungs (con. unit) | Seeding M.tub. (CFU) |
| Control group | 45% | 3.24 | 4.5 | 300 |
| Streptomycin | 0 | 2.47 | 2.45 | 200 |
| R₁ = R₂ = Cl | 0 | 2.42 | 2.5 | 195 |
| R₁ = R₂ = Br | 0 | 2.30 | 2.3 | 180 |
| CFU - are colony forming units | | | | |

The results of the investigations conducted show that in experimental animals with generalized tuberculosis process, the claimed compounds showed a therapeutic effect, which as to the exhibited activity, is not less than that of streptomycin.

### Experiment 5

### Determination of Action of Claimed Compounds on Herpes Virus

Antiviral activity was studied relative to the herpes virus I of the type (VPG-1/Leningrad/248/88) in accordance with a well known method (21). The viruses were grown on inoculated culture of cells Vero, obtained from the bank of cellular cultures of Institute of Cytology RAS.

### Procedure

Virus in a final concentration 10 part/ml and the claimed compounds diluted in DMSO with final concentration 100, 10 and 1 mg/l were added to cells which had been grown on RPMI-1640 medium with 10% bovine serum, and introduced into wells of 96-wells plate. For each tested concentration of the compound, five (5) independent holes were used. The plate was incubated for 60 min at 38°C in a CO₂ incubator. After the incubation the virus was withdrawn and again fresh medium was introduced containing the claimed compounds in used concentrations.

The results were evaluated as to the presence of cytopathogenic action of virus on the cells after 36 hours of incubation at 38°C in the CO₂ incubator.

The following controls were used in the experiment:
1. Control of cell culture (ability to normal growth);
2. Control of virus (evaluation of ability to reproduction);
3. Control of antiviral activity of antiviral compound acyclovir;
4. Control of compounds (toxicity of compounds); and
5. Control of solvent (DMSO) as to toxicity.

For evaluation of cytopathic action of virus, the number of unchanged cells was calculated in 100 fields formed by a special net of an eyepiece of an inverted microscope. The results obtained are presented in Table 9.

**Table 9**

| Action of Claimed Compounds to Herpes of Simplex Virus | | | | |
|---|---|---|---|---|
| NN | Compound | Concentration of Tested Co pounds(mg/l) | | |
| | | 100 | 50 | 10 |
| 1. | Acyclovir | -* | - | 8000**(80%)*** |
| 2. | DMSO | 10000 | 10000 | 10000 |
| 3. | R₁ = H, R₂ = NO₂ | toxic | toxic | 6000 (60%) |
| 4. | R₁ = R₂ = NO₂ | toxic | 5000 (50%) | 4000 (40%) |
| 5. | R₁ = R₂ = H | toxic | 6000 (60%) | 3000 (30%) |
| 6. | Control of Cells | 10000 | 10000 | 10000 |

| | | | | |
|---|---|---|---|---|
| * compound of given concentration which is not tested | | | | |
| ** number of cells in 100 fields under consideration | | | | |
| *** in the brackets the percentage of protection of cells from virus when compared with the control of cell cultures is presented. | | | | |

These results show that the claimed compounds presented in Table 9 possess anti-herpes activity which is comparable with that of acyclovir, which is used standardly to treat herpes. The remaining claimed compounds had less pronounced activity in the process of suppression of reproduction of herpes virus in the selected conditions of experiment

### Experiment 6

### Determination of Interferon-Inducing Activity of Claimed Compounds

Induction of the synthesis of interferons with the claimed compounds was performed on the primary culture of human lymphocytes (in particular these cells in the human organism are main producers of interferons). In order to obtain the culture of lymphocytes, fresh (12 hours after taking) blood of healthy donors (not of second group) was used. In order to separate lymphocytes, heparin-treated blood obtained from a healthy donor was subjected to centrifuging in a gradient of density ficoll-verographin 1.71 g/cm³ for separation of fraction of immuno-competent cells. This fraction was selected and reproduced by a nutrient medium RPMl-1640 containing 5% of fetal calf serum, 0.3 mg/ml of L-glutamine, 100 unit/ml of penicillin, 50 g/ml of streptomycin. Concentration of lymphocytes was considered after covering with Methylene Blue and counting of the number of cells in the Goraev chamber. The initial solvents of the claimed substances were diluted by the nutrient medium RPMI-1640 so that the final concentration of substances will form a series: 100 mg/l, 10mg/l, 1 mg/l after introduction of lymphocyte suspension. The final concentration of lymphocytes in the induction mixture was 3x10⁶ cell/ml. Parallel to the test samples the following controls were presented:
1) Control of spontaneous production of interferons (IFN) by lymphocytes;
2) Control of process flow under the action of standard IFN inductor N-metyl-N-(α, D-glucopyranosyl)-ammonium-10-methylene carboxylate acrydone (cycloferon);
3) Control of process flow under the action of standard IFN Neovir (sodium 10-methylenecarboxylate-9-acrydone) with corresponding content of DMSO in test samples; and
4) Control of spontaneous production of interferons in the presence of DMSO, in the quantity corresponding to tested samples.

Control and test samples were incubated for 24 hours at 37°C. After the incubation the samples were centrifuged at 2000 G for sedimentation of cellular elements. The IFN-containing supernatant was then withdrawn and analyzed to determine the quantitative content of IFN. The sediment of the cells was resuspended in the former volume of nutrient medium, colored with vital coloring-trypan Blue, and the number of cells was counted in the Goraev chamber (as described above) for determination of cytotoxic action of the compounds.

The quantitative determination of IFN content in control and test samples was performed with the use an immuno-ferment test system for IFN produced by TOO "protein contour" Pro Con If2 plus. In order to determine the quantity of interferon in the sample solid, the phase immuno-ferment method was used and horseradish peroxidase was used as an indicator ferment. Activity of bonded peroxides was measured with the use of an automatic photometer for microplates with a microprocessor at the wavelength of 450 nm.

For counting the results, the activity of IFN in standard solutions of IFN containing the known quantity of compound was determined at the same time. Based on the obtained results, a calibrating curve was formed, which permits, with the use of the microprocessor of the automatic photometer, obtaining data expressed in international units of activity (IU). The results of analysis are expressed in IU activity of IFN per ml in the given induction system, which contains 3x10⁶ of lymphocytes in ml. Each test and control point was investigated in four parallels.

### Controls of immunoferment reaction.

1. Control DMSO with a nutrient medium.
2. Control of system components (in accordance with instruction).
3. All results were considered only when there was a correspondence of the controls to the passport data of the system.

The results obtained were subjected to statistical analysis in accordance with t-criterion and calculation of confident interval at p=0.05. The analysis of coincidence of results in the parallel tests was performed. As a result of the performed investigations, it was determined that among the claimed compounds, there are samples which have the capability of inducing the synthesis of IFN (Table 10).

**Table 10**

| Quantitative Evaluation of IFN Inducing Activity of Claimed Compounds | | | | |
|---|---|---|---|---|
| N | Compound | Content of IFN in induction mixture after 24 hours of incubation at various concentrations of compounds (µg/ml), ME/3*10⁴lymph./ml | | |
| | | 100 | 10 | 1 |
| 1. | Control of Lymphocytes | 0 | 0 | 0 |
| 2. | Cycloferon | 58 ± 1.4 | 22 ± 2.5 | 3.8 ± 0.8 |
| 3. | Neovir | 66 ± 1.4 | 24.5 ± 1.2 | 4.1 ± 0.5 |
| 4. | Poly I/ poly C | -* | 43.6 ± 2.0 | 10.5 ± 0.8 |
| 5. | DMS0 | 0 | 0 | 0 |
| 6. | R₁ = H, R₂ = NO₂ | 48 ± 1.2 | 20 ± 1.2 | 4.2 ± 0.8 |
| 7. | R₁ = Cl, R₂ = NO₂ | 36 ± 1.4 | 18 ± 1.6 | 3.6 ± 0.5 |
| 8. | R₁ = H, R₂ = Br | 192 ± 1.6 | 84 ± 2.5 | 14 ± 0.5 |
| 9. | R₁ = R₂ = Br. | 47 ± 0.8 | 23 ± 1.0 | 4.0 ± 0.5 |

| | | | | |
|---|---|---|---|---|
| * - is a compound of given concentration which was not tested | | | | |

### Experiment 7

### Determination of Action of Claimed Compounds to Chlamydia Trachomatis

Antimicrobial activity of claimed compounds was studied relative to *C. trachomatis* D323, which is a standard strain from the collection of the chair of microbiology of St. Petersburg (Russia) State Pavlov Medical University. This strain was derived from a patient with Chlamydia urethritis and has a morphology and physiological activity which is characteristic for the representatives of this type. It is sensitive to action of compounds used for treatment of a chlamydia infection.

Cell cultures McCoy and L929 obtained from the Institute of cytology of RAS were used in this work.

### Procedure of Experiment

Cells were grown in flasks from neutral glass in the medium RPMI-1640 with the addition of 10% of fetal bovine serum. The test was performed in glass (non toxicity) flat-bottom flasks with cover glasses. The cells were introduced into the medium in the final concentration 1x10 cell/ml. After obtaining of monolayer, in the test tubes, standard infectious dosages of chlamydia were introduced. The dosages of chlamydia had been stored in frozen condition at -70° C. Simultaneously, compounds in the final concentration 100 mg/l were added to the cells. The samples were centrifuged at 2400 G for 60 minutes at room temperature and incubated at 37°C for 2 hours. The nutrient medium in the flasks was then exchanged for new medium containing 5% embryo bovine serum and cycloheximide (2 mg/ml), with a repeated introduction of the claimed compounds in the same concentration. At the same time, the samples were doubled with the use of the medium without cycloheximide so as to exclude its action on the investigated substances. The samples were then incubated for 48 hours in CO₂ incubator.

The controls included: control of cell cultures; control of action of solvents; control of action of chlamydia in the absence of any compounds; control of sensitivity of chlamydia to standard antimicrobial compound ciprofloxacin (19); and control of tested compounds as to toxicity relative to cell cultures.

The evaluation of results was performed by determination of chlamydia cytoplasmatic inclusions by means of the method of immunofluorescence (MicroTrac *Chlamydia trachomatis* Direct Specimen Test) and chlamydia antigens by means of CylaMonoScreen (Russian-British Joint Venture, 66 Regent's Park Road London NW1 7SX) (22, 23). The effect of action of the claimed compounds was determined by analysis of the condition of the monolayer and the number of cells with CPV relative to the control (cell culture infected with *C. trachomatis* D323). Also considered was the number of normal cells in 100 vision fields obtained with the use of a special net of the eyepiece of a microscope.

The results of control samples which satisfied the requirements of the experiment were: control of cell culture-morphology of cells and condition of monolayer correspond to the given type of cells; control of chlamydia growth in the cell culture-presence of CPV in the monolayer; and control of action of standard antimicrobial compound determination of the reduction of numbers of CPV in the monolayer when compared with the proceeding control. The controls of the toxicity of the claimed compounds and the toxic action of solvents on the cells are absent. The results of tests performed are set forth in Table 11.

**Table 11**

| Action of Claimed Substances on C.trachomatis | | |
|---|---|---|
| N | Substance | Number of unchanged cells |
| 1 | Cell control | 8.000 |
| 2. | DMSO | 8.000 |
| 3. | Control of infected cells | 6.000 |
| 4. | Ciprofloxacin (100)* | 7.000 (50%)** |
| 5. | R₁ = H, R₂ = Br (100) | 7.000 (50%) |
| 6. | R₁ = H, R₂ = H (10) | 7.200 (60%) |

| | | |
|---|---|---|
| * concentration of compound in mg/l is given in brackets | | |
| ** percent of protection of cells from infection is given in brackets. | | |

The data set forth in Table 11 proves that the claimed compounds possess pronounced activity against chlamydia which exceeds that of the ciprofloxacine, the standard compound used to treat chlamydia.

The remaining claimed compounds have less pronounced activity as to protection of cells from chlamydia in the given conditions of experiment.

### Industrial Applicability

Examples 1-5 and results of practical synthesis and analysis of claimed compounds presented in the Tables 1-4 prove the possibility of laboratory and industrial synthesis of all 10 claimed compounds by means managed with modem pharmaceutical industry, and also their clear identification by widely used control methods.

The series of experiments for determination of biological activity presented in seven reports showed that the claimed compounds possess biological activity to various microorganisms including mycobacteria (sensitive and stable to existing compounds), chlamydia, Herpes Simplex viruses, and also interferon-inducing activity. The latter indicates the possibility of their use for treatment of some cancer diseases.

The presented facts show the achievement of the objectives posed by the invention: a new class of heterocycic compounds was synthesized which possess high and broad biological activity, particularly anti-mycobacteria (causing tuberculosis and mycobacteriosis), immunostimulating, antichlamydial and antiviral activity.

Therefore, in accordance with our opinion, the claimed agents (compounds) satisfy all requirements for an invention: they are new, unobvious, and industrially applicable.

### Literature

1. Grosset, J., Current Problems With Tuberculosis treatment, Res. Microbiology, 1996, Vol. 147, No. 10-16.
2. Dyson, G.M., May P. Chemistry of Synthetic Medications - translation from English, Moscow, Mir, 1964, pages 660.
3. Bockmuhl, Persch, U.S. patent No. 2,323,445.
4. Bernheim, H., J. of Bacteriology, 1941, Vol. 41, P. 387.
5. Pharmaceutical Microbiology, Ed. By W.B. Hugo and A.D. Russel Blackwell Scientific Publications, Oxford, 1987, 511p.
6. Benson, C.A. Treatment of Disseminated Mycobacterium Avium Complex Disease: A Clinician's Perspective, Res. Microbiology, 1996,Vol. 147, P. 16-24.
7. Saltzman, R., Jurewicz R., Boon B, Safety of Famciclovir in Patients with Herpes Zoster and Genital Herpes, Antimicrobial agents and Chemotherapy, 1994, Vol. 38, No. 10, P. 2454-2457.
8. Fenelon, L. E., Mumtaz G., Ridgway G.L. The *In-Vitro* Susceptibility of *Chlamydia Pneumoniae*, J. of Antimicrobial Chemotherapy, 1990, Vol. 26, P. 763-767.
9. Boyd, M.R. The Future of New Drug Development. Current Therapy In Oncology 1992, P. 11-22.
10. Negwer, M. Organic-Chemical Drugs and Their Synonyms, Akademic-Verlag, Berlin, 1987.
11. Biswas, C. Dissert. Abstracts 1964, Vol. 24, No. 9, P. 3501.
12. Langley, B.W., British patent no. 845378 of 08.24.60.
13. Spasov, A.B., and Raikov, Z.D., inventor's certificate of Bulgaria No. 17122, int.cl C07D 51/22, applied 06.05.71.
14. Davies, G.D., Robins, P., Cheng, CC., J. of American Chemical Society, 1962, Vol. 82, No. 9, P.1724-1729.
15. Minchle, H., Milzher, K, Keisser, F., German application no. 2 412 854, int. C07D of 09.26.1974.
16. Krepelka, J., Kotva, R., Pijman, V., Semonsky, M., patents of Czechoslovakia no. 2155554; 215593; 215580, int C07D 239/54 of 04.15.1984.
17. Ueda, T., Kato S., Japanese patents 11832 ('62)1 08.23., applied 05.10.1958.
18. Sigao, S., Hirosi, I., J. of Pharmaceutical Society of Japan, 1969, Vol.,89, No. 2, P. 266-271.
19. Senda, S., Fujimura, H., Izumi, I., Japanese patent no. 7912; 7913;7914; 7915; 7916; April 22, applied 10.05.1961.
20. Blythin, D.J., Coldwell N.J., U.S. Patent No. 4,272,535 published 07.09.1981, applied 07.27.1979.
21. Gentry, G.A., Lawrency, N., Lushbaugh, N. Isolation and Differentiation of Herpes Simplex Virus and Trichomonas Vaginalis in Cell Culture, J. of Clinical Microbiology 1985, Vol. 22, No. 2, P. 199-204.
22. Wang, S-P., Grayston J.T., Serotyping of *Chlamydia trachomatis* by Indirect Fluorescent-Antibody Staining of Inclusions in Cell Culture with Monoclonal Antibodies, J. of Clinical Microbiology, 1991, Vol. 29, No. 7, P. 1295-1298.
23. Judson, BA., Lambert, P.P. Improved Syva Micro Trac *Chlamydia Trachomatis* Direct Test Method, Journal of Clinical-Microbiology, 1988, Vol. 26, No. 12, P. 2657-2658.

## Claims

1. Biologically active substance on the basis of tetracyclic heterocycles of pyrimidine series, characterized in that it represents a derivative of 5-oxo-5H-[1]-benzopyrano-[5,6-b]-4-oxo-4H-[1,2]-pyrimido-1,4,5,6-tetrahydro-1,3-thiazine (1) of general formula (1) 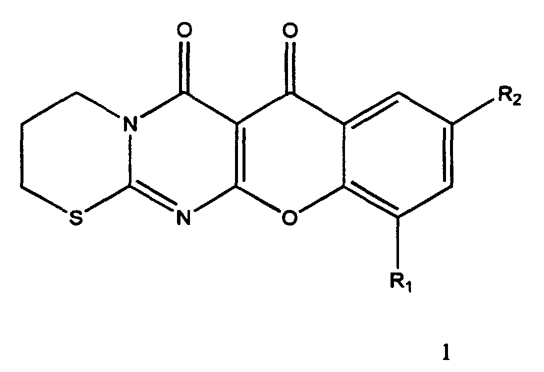 where: R₁ = H or a halogen; R₂ = H, halogen, nitro, hydroxy or methoxy.

2. Compound in accordance with claim 1, characterized in that R₁ = R₂ = H (I).

3. Compound in accordance with claim 1, characterized in that R₁ = H and R₂ = Cl (II).

4. Compound in accordance with claim 1, characterized in that R₁ = R₂ = Cl (III).

5. Compound in accordance with claim 1, charactarized in that R₁ = H and R₂ = Br (IV).

6. Compound in accordance with claim 1, characterized in that R₁ = R₂ = Br (V).

7. Compound in accordance with claim 1, characterized in that R₁ = H and R₂ = NO₂ (VI).

8. Compound in accordance with claim 1, characterized in that R₁ = Cl and R₂ = NO₂ (VII).

9. Compound in accordance with claim 1, characterized in that R₁ = Br and R₂ = NO₂ (VIII).

10. Compound in accordance with claim 1, characterized in that R₁ = H and R₂ = OCH₃ (IX).

11. Compound in accordance with claim 1, characterized in that R₁ = H and R₂ = OH (X).
